# EUROPÄISCHE PATENTSCHRIFT

(11) **EP 2 518 436 B1**
(45) Veröffentlichungstag und Bekanntmachung des Hinweises auf die Patenterteilung: **17.06.2015**
(21) Anmeldenummer: 12002841.0
(22) Anmeldetag: 23.04.2012
(51) Int. Cl.: G01B 7/16

(54) **Biegesensor**
Bend sensor
Capteur de flexion

(30) Priorität: 28.04.2011 DE 102011017704
(43) Veröffentlichungstag der Anmeldung: 31.10.2012
(73) Patentinhaber: Storz Endoskop Produktions GmbH, 78532 Tuttlingen (DE)
(72) Erfinder: Krattiger, Beat, 78532 Tuttlingen (DE); Wagner, Sebastian, 78532 Tuttlingen (DE); Carrard, Jérome, 78532 Tuttlingen (DE); Krämer, Sina, 66583 Spiesen-Elversberg (DE)

(56) Entgegenhaltungen:
- DE-A1-102006 027 616
- US-A- 5 379 644

## Beschreibung

Die Erfindung betrifft einen Biegesensor zur Messung einer Auslenkung bestehend aus einem lang erstreckten Körper aus elektrisch isolierendem Polymermaterial mit in den Körper eingebetteten Fasern aus einem elektrisch leitenden Polymermaterial.

Die Erfindung betrifft ferner ein medizinisches und/oder endoskopisches Instrument und einen medizinischen Haltearm mit einem Biegesensor der vorstehend genannten Art.

Biegesensoren finden in vielen Gebieten Anwendung um die Auslenkung eines Objekts aus seiner Längsachse zu bestimmen und verwenden häufig elektrisch leitende Materialien um über eine Änderung von Widerstand, Kapazität oder Induktivität die Richtung oder Stärke der Auslenkung messbar zu machen. Die biegbaren oder auslenkbaren Objekte sind beispielsweise technische oder medizinische Instrumente. Wann immer zwei Abschnitte eines Gegenstands einen veränderlichen Winkel miteinander bilden, können Biegesensoren Anwendung finden um die Biegung zu messen.

In der Medizintechnik und medizinischen oder technischen Endoskopie werden Biegesensoren auch verwendet um die Lage und Auslenkung von flexiblen oder abwinkelbaren Instrumenten zu bestimmen. Insbesondere bei flexiblen Endoskopen ist es üblich geworden das distale Ende so auszugestalten, dass es über eine Handsteuerung oder motorgetrieben ausgelenkt werden kann. Um in engen Räumen und Körperhöhlen das Instrument genau steuern zu können, soll die Auslenkung des Instrumenteneinführteils, insbesondere des Distalendes, möglichst genau erfasst werden. Dabei ist es erforderlich, dass die verwendeten Biegesensoren die Auslenkung entlang einer X- und Y-Achse messen. Da die Instrumente häufig einen kleinen Durchmesser haben und darin bereits Lichtleiter, Bildleiter, Arbeitskanäle, Bowdenzüge oder dergleichen untergebracht sind, dürfen auch die Biegesensoren und deren Kontakte und die erforderlichen elektrischen Leitungen nur wenig Raum einnehmen.

Aus der US 6,201,468 B1 ist ein Biegesensor zur Verwendung in Joysticks bekannt, mit einem elastischen und elektrisch isolierenden Grundkörper, in den ein zentraler Strang und mehrere umliegende Stränge aus leitendem Gummi eingelassen sind. Zwischen den umliegenden und dem zentralen Strang befindet sich jeweils eine Lücke, die erst bei Biegung des Sensors sukzessive geschlossen wird. Die Kontaktfläche zwischen umliegendem und zentralem Strang und der dabei gemessene elektrische Widerstand korrespondieren mit der Richtung und dem Grad der Biegung des Sensors.

Nachteilig an diesem bekannten Biegesensor ist, dass zwischen den Strängen Lücken bleiben müssen, was zu einem größeren Sensor führt und bei kleiner Bauform eine besonders exakte Fertigung erfordert. Auch gibt das über die Kontaktfläche erzeugte Signal die Biegung des Sensors insbesondere bei großen Biegewinkeln nicht genau wieder.

Aus der US 5,728,044 A ist ein flexibler Stab zur Einführung in Arbeitskanäle flexibler medizinischer Instrumente bekannt, auf dessen Oberfläche eine Vielzahl von Biegesensoren befestigt ist, um eine Auslenkung des medizinischen Instruments und damit des Stabs anhand der von den Sensoren erzeugten elektrischen Signale zu bestimmen.

Diese Konstruktion hat den Nachteil, dass mehrere Sensoren verwendet werden müssen um Bewegungen in verschiedenen Raumrichtungen zu erfassen. Für jeden Sensor sind eigene elektrische Leitungen notwendig, die außerdem Platz benötigen. Auch sind die verwendeten flachen Biegesensoren nur dazu geeignet, Biegungen in eine Richtung zu messen, der Sensor lässt sich nicht in Richtung seiner schmalen Längsseite biegen. Sie sind auch weniger flexibel als der Stab auf dem sie angebracht sind, was zu fehlerhaften Messsignalen führt. Des Weiteren ist dieser bekannte Stab nicht gegen Torsion geschützt, die die Messung der Auslenkung beeinträchtigt. Es sind zusätzliche Torsionssensoren notwendig, um eine mögliche Verdrehung des Stabes zu berücksichtigen.

Die DE102006027616A1 beschreibt Dehnungsmessstreifen auf Basis extrinsisch elektrisch leitfähiger Polymerwerkstoffe. Durch das Füllen mit Metallfasern wird der Polymerwerkstoff elektrisch leitfähig. Diese Messgitterfasern können linear angeordnet sein. Der gefüllte Polymerwerkstoff kann als Leiterbahn auf einer Trägerfolie aufgebracht sein.

Die US 5,379,644 beschreibt Dehnungsmessstreifen aus elektrisch leitenden Carbonfaserbündeln, die mit einem Kunstharz umgeben und in Bauteile eingebracht werden können, um dort Belastungen zu messen.

Davon ausgehend liegt der Erfindung die Aufgabe zugrunde, einen verbesserten Biegesensor zu schaffen, der einfach zu fertigen ist und eine genaue Messung ermöglicht. Es ist weiterhin eine Aufgabe der Erfindung, ein medizinisches und/oder endoskopisches Instrument oder einen medizinischen Haltearm mit einem verbesserten Biegesensor zu schaffen, so dass eine Auslenkung genau erfasst werden kann.

Die Begriffe Krümmung, Biegung, Abwinkelung und Auslenkung werden alternativ verwendet und bezeichnen die Biegung des Sensors bzw. des betreffenden Gegenstands, an dem der Sensor angebracht ist, aus seiner Längsachse heraus. Es versteht sich, dass auch die Differenz zwischen zwei ausgelenkten bzw. gebogenen Zuständen auf diese Weise bezeichnet sein kann.

Erfindungsgemäß werden diese Aufgaben durch die Merkmale der Patentansprüche 1, 14 oder 15 gelöst. Vorteilhafte Weiterbildungen sind Gegenstand der abhängigen Ansprüche.

Ein erfindungsgemäßer Biegesensor weist einen lang erstreckten Körper aus elektrisch isolierendem Polymermaterial auf, in den Fasern aus elektrisch leitendem Polymermaterial eingebettet sind. Die Fasern sind im Wesentlichen parallel zueinander und voneinander beabstandet im Körper angeordnet. Die Zwischenräume zwischen den Fasern werden vom isolierenden Polymermaterial ausgefüllt. Der Biegesensor weist vorzugsweise einen Querschnitt auf, der annähernd rund oder auch quadratisch ist oder eine andere Form hat, die zumindest entlang ihrer Symmetrieachsen jeweils eine ähnliche Ausdehnung aufweist und so eine flexible und gleichmäßige Biegung des Sensors in viele Richtungen ermöglicht ohne eine Richtung aufgrund der Geometrie zu bevorzugen.

Durch Bestimmung der Änderung der elektrischen Widerstände zweier Fasern mit Hilfe einer Messeinheit, die mit den Fasern verbunden ist, kann eine Auslenkung des Sensors aus seiner Längsachse in der Ebene, in der die beiden Fasern liegen, leicht und zuverlässig erfasst werden. Die Änderung der elektrischen Widerstände der Fasern ist dabei etwa proportional zum Grad der Auslenkung und kann genau gemessen werden. Das Polymer des Körpers und das Polymer der Fasern kann ein Elastomer, bspw. ein Silikon oder dergleichen, sein. Die Flexibilität des Sensorkörpers ist bevorzugterweise vergleichbar mit der der Fasern. Die Fasern sind durch den Körper voneinander getrennt und berühren sich nicht.

Ein solcher erfindungsgemäßer Biegesensor mit Polymerkörper und ganz oder teilweise eingebetteten Fasern kann zudem leicht durch Coextrusion und in beliebiger Länge hergestellt werden. Der Querschnitt der Fasern kann dabei eine beliebige Form annehmen und beispielsweise rund oder oval sein oder die Form eines Kreissegments haben.

Durch die einfache und kostengünstige Ausgestaltung des erfindungsgemäßen Biegesensors ist eine Vielzahl von Anwendungen denkbar. So kann er auch als redundanter Sensor zu einem hoch präzisen Biegesensor in der Robotik Anwendung finden, und dort für zusätzliche Sicherheit sorgen. Auch die Anwendung in der Biomechanik ist denkbar, um die Bewegung lebender Körper zu messen und nachzuvollziehen.

In einer bevorzugten Ausgestaltung der Erfindung weist der erfindungsgemäße Biegesensor wenigstens vier Fasern auf, die umfänglich um etwa 90° zueinander versetzt angeordnet sind. Mit vier so angebrachten Fasern ist es möglich eine Auslenkung sowohl entlang einer zur Längsachse des Biegesensors senkrechten X- als auch einer dazu orthogonalen Y-Achse in jeweils zwei entgegengesetzten Richtungen zu erfassen. Selbstverständlich werden dadurch auch alle sonstigen Biegungen korrekt gemessen, die sich in ihrer Richtung aus einem X- und einem Y-Anteil zusammensetzen. Es ist nicht notwendig mehrere Sensoren zu verwenden, um alle diese Richtungen zu erfassen. Der Biegesensor kann aufgrund seiner erfindungsgemäßen Ausgestaltung auch Krümmungen mit einem großen Biegewinkel von mehr als 180° richtig messen.

Gemäß einer weiteren vorteilhaften Ausgestaltung sind am Biegesensor Mittel zur Versteifung des Sensors vorgesehen, die eine Verdrehung des Biegesensors um seine Längsachse beschränken oder verhindern. Diese können alle Mittel sein, die die Torsion vollständig oder nahezu vollständig unterbinden, jedoch gleichzeitig die Biegung des Sensorkörpers nicht beeinträchtigen.

Vorteilhafterweise ist der Biegesensor hierfür entlang seiner Längsachse zumindest teilweise von einem an seinem Körper anliegenden Flechtschlauch umschlossen. Der Flechtschlauch kann erfindungsgemäß aus einem Polyamid, beispielsweise aus Nylonfasern, aus Glas, Kohlenstoff oder aus Polyaramid bestehen und auf den Körper aufgebracht bzw. auch auf den Körper aufgeklebt sein. Auch kann es sich bei dem erfindungsgemäßen Flechtschlauch um eine Flechtschicht handeln, die bei der Herstellung durch Coextrusion gleich mit auf den Sensor aufgebracht wird und kein separates Teil darstellt.

Durch den Flechtschlauch wird erfolgreich verhindert, dass der Biegesensor um seine Längsachse verdreht wird. Dies macht aufwändige Torsionssensoren oder dergleichen, die eine ungewollte Verdrehung erfassen um eine Korrektur des Messsignals ermöglichen, überflüssig. So kann mit einer einfachen und preiswerten Maßnahme eine korrekte Funktionsweise des Biegesensors sichergestellt werden. Der Flechtschlauch hat darüber hinaus die Eigenschaft, den Sensorkörper vor mechanischer Beanspruchung zu schützen und/oder ihn nach außen hin dort elektrisch zu isolieren, wo keine elektrische Verbindung mit anderen Bauteilen gewünscht ist.

In einer bevorzugten Ausführung ist der Durchmesser des Biegesensors über seine gesamte Länge im Wesentlichen kleiner als ein Millimeter. Durch die spezielle Ausgestaltung des Biegesensors ist es möglich ihn in so kleinen Dimensionen herzustellen, dass er bequem in verschiedene medizinische und/oder endoskopische Instrumente integriert werden kann.

Nur mit diesem kleinen Durchmesser ist es auch möglich, den Biegesensor beispielsweise in flexiblen Endoskopen zu verwenden, wo im Schaft aufgrund der vielen anderen bereits vorhandenen Komponenten wie Glasfasern oder Arbeitskanäle nur wenig Platz zur Verfügung steht. Insbesondere in der Medizintechnik müssen die Instrumente durch enge Körperöffnungen eingeführt werden, so dass im Schaftquerschnitt nur wenige Quadrat-Millimeter zur Verfügung stehen.

Gemäß einer weiteren bevorzugten Ausgestaltung sind elektrische Leitungen vorgesehen, die die Fasern im Bereich der Faserenden kontaktieren. Diese Leitungen stellen eine elektrische Verbindung der Fasern untereinander und mit der Messeinheit her, um die Widerstandsänderung in den Fasern als Maß für die Auslenkung zu erfassen.

Dabei bilden vorzugsweise jeweils zwei der Fasern und die elektrischen Leitungen zusammen mit der Messeinheit eine elektrische Schaltung, die als Wheatstone'sche Brücke bezeichnet wird und dem Fachmann bekannt ist. Die veränderlichen elektrischen Widerstände werden dabei durch die Fasern gebildet. Die notwendigen Referenzwiderstände sind beispielsweise in der Messeinheit untergebracht. Damit ist die Detektion einer Auslenkung des Biegesensors aus seiner Längsachse möglich. Für die gewünschte Erfassung von Auslenkungen entlang der zur Längsachse des Sensors senkrechten X- und Y-Achse werden zwei derartige Schaltungen kombiniert und die Fasern entsprechend elektrisch kontaktiert. Durch die Auslagerung der Referenzwiderstände in eine Messeinheit kann im Sensor und in der Vorrichtung, in der der Sensor Anwendung findet, Platz gespart werden.

Nach einer weiteren vorteilhaften Ausgestaltung des Biegesensors liegen die Fasern wenigstens teilweise an der Oberfläche des Körpers. Bei dieser Anordnung ist es besonders leicht die Fasern elektrisch zu kontaktieren, da sie nach außen nicht vollständig durch den Polymerkörper elektrisch isoliert werden.

Gemäß einem weiteren vorteilhaften Ausführungsbeispiel sind die elektrischen Leitungen zumindest teilweise in das elektrisch isolierende Polymermaterial des Körpers eingebettet. Dies hat nun den Vorteil, dass die Leitungen innerhalb des Körpers geführt werden und somit den Durchmesser des Sensors nicht vergrößern. Eine besonders kompakte Bauweise wird dadurch ermöglicht.

Die elektrischen Leitungen können beispielsweise Drähte sein, die durch den Körper geführt werden und jeweils vorzugsweise an den Faserenden entsprechend mit den Fasern verbunden werden.

Noch vorteilhafter können die elektrischen Leitungen zusätzliche in den Körper eingebettete Fasern aus dem leitenden Polymermaterial umfassen. Diese sind von den zur Erfassung der Biegung verwendeten Fasern durch den Körper größtenteils elektrisch isoliert und werden nur an den Faserenden mit diesen elektrisch verschaltet. Erfindungsgemäß kann ein solcher Sensorkörper mit allen notwendigen Polymerfasern in einem Stück coextrudiert und somit besonders einfach hergestellt werden.

Die elektrische Verbindung an den Faserenden kann vorteilhafterweise durch Drähte oder Mikronägel hergestellt werden, die in die Fasern eingestochen werden und entsprechend der Schaltung die verschiedenen Fasern miteinander verbinden. Auch verschiedene andere Arten der Kontaktierung sind denkbar. Wenn die Fasern teilweise an der Oberfläche des Körpers liegen, kann die Kontaktierung über die Außenseite des Sensors erfolgen.

In einer weiteren besonders geeigneten Ausführungsform ist der Körper entlang seiner Längsachse mit einem Kanal versehen, der beispielsweise mittig durch den Körper verlaufen kann, wobei die elektrischen Leitungen zumindest teilweise durch den Kanal geführt werden. Auf diese Weise können die Leitungen auch nach Herstellung des Sensorkörpers noch auf einfache Weise und platzsparend verlegt werden.

Eine besonders einfache und dem erfindungsgemäß kleinen Durchmesser des Biegesensors angepasste Kontaktierung erfolgt gemäß einer weiteren Ausgestaltung über Flexprints. Das Wort Flexprint bezeichnet flexible Leiterplatten mit gedruckten Leiterbahnen. Diese Leiterplatten bestehen beispielsweise aus einem flexiblen Kunststoff und lassen sich gut formen, um den Biegesensor an den Faserenden ganz oder teilweise zu umschließen. An den Flexprints vorgesehene Kontakte werden der Schaltung entsprechend direkt mit solchen Fasern verbunden, die teilweise an der Oberfläche des Körpers liegen, ansonsten jedoch in den Sensorkörper eingebettet sind. Teile der Flexprints können auch durch den Kanal zum proximalen Ende des Sensors geführt werden, wo sie mit entsprechenden Zuleitungen zur Messeinheit verbunden werden.

Die Fasern und/oder die Flexprints sind in einer erfindungsgemäßen Weiterbildung des Biegesensors mit Vertiefungen versehen, in welche Vorsprünge der jeweils anderen Komponente Flexprint oder Faser eingreifen, um eine sichere Kontaktierung zu gewährleisten und ein Verrutschen zu vermeiden, und außerdem die Flexprints möglichst eng am Biegesensor anliegen zu lassen

In einer weiteren vorteilhaften Ausgestaltung ist am Sensor außen ein Schrumpfschlauch vorgesehen, der den Biegesensor über die ganze Länge oder teilweise umschließt, um alle Komponenten des Biegesensors, wie Sensorkörper, Flechtschlauch und elektrische Leitungen in der richtigen Position zu halten und ein Verrutschen auch bei Auslenkung des Biegesensors zu vermeiden. Auch kann es sich um zwei oder mehrere Schrumpfschlauchteile handeln, die insbesondere jeweils an den Enden des Sensors angebracht werden. Dies stellt ein platzsparendes Mittel dar, um die Komponenten sicher aneinander zu halten. Um bei der Fertigung die korrekte Kontaktierung einfach prüfen zu können, ist der Schrumpfschlauch vorzugsweise transparent ausgebildet.

Aufgrund seiner kompakten Bauform und möglichen kleinen Abmessungen ist der erfindungsgemäße Biegesensor besonders geeignet, in medizinischen und/oder endoskopischen Instrumenten, beispielsweise flexiblen Endoskopen, angewendet zu werden. Ein erfindungsgemäßes Instrument weist einen Einführteil auf, mit wenigstens einem flexibel ausgebildeten Abschnitt, wobei der Biegesensor im Bereich dieses flexiblen Abschnitts im Einführteil angeordnet ist, um eine Auslenkung des Einführteils mit Hilfe des Biegesensors zu messen.

Auch in anderen Instrumenten, die aufgrund ihrer Anforderungen einen Schaft mit geringem Durchmesser aufweisen, wie beispielsweise flexible Fasszangen, Katheter oder andere medizinische Instrumente, kann der Biegesensor vorteilhaft eingesetzt werden und eine genaue Kontrolle der Schaftauslenkung ermöglichen. Selbstverständlich können auch mehrere flexible Abschnitte am Einführteil vorgesehen sein, die jeweils mit einem Biegesensor versehen sind.

Nach einer weiteren vorteilhaften Ausgestaltung ist der Biegesensor dabei vollständig vom Einführteil umschlossen. Auf diese Weise kann der äußere Aufbau des Einführteils des Instruments gleich bleiben, der beispielsweise verschiedene Schläuche, Netzgeflechte und Bowdenzüge umfasst. Es muss lediglich im Inneren des Einführteils Raum für den Biegesensor vorgesehen werden, was aufgrund seiner erfindungsgemäß kleinen Abmessungen möglich wird. Dies ermöglicht ein einfaches modulares Nachrüsten des Sensors in bestehende Instrumente, ohne dass aufwendige Umkonstruktionen der Instrumente nötig werden. Der Sensor ist im Schaft sicher und geschützt untergebracht. Die elektrischen Leitungen werden dabei bevorzugt vom Biegesensor durch den Einführteil zum proximalen Ende des Instruments geführt, wo sie mit der Messeinheit verbunden sind.

In einer weiteren vorteilhaften Ausgestaltung eines erfindungsgemäßen medizinischen und/oder endoskopischen Instruments ist eine Motoreinheit vorgesehen, die mit dem flexiblen Abschnitt des Instruments in Verbindung steht um eine Auslenkung des Einführteils zu bewirken. Die Messeinheit und die Motoreinheit sind hierbei über Signalleitungen zur Übertragung eines Signals als Maß der Auslenkung miteinander verbunden.

Dies hat nun den Vorteil, dass eine motorisch bewirkte Auslenkung des flexiblen Abschnitts gemessen werden kann, und dass die von der Messeinheit erzeugten Signale durch Rückmeldung an die Motoreinheit verwendet werden können, um die motorische Auslenkung verbessert steuern zu können. Die ausgelenkte Lage des medizinischen und/oder endoskopischen Instruments wird dabei genau erfasst und liefert eine Information darüber, ob die motorische Auslenkung korrigiert werden muss. Dies stellt eine erhebliche Verbesserung des gesamten Instruments dar, da ein präziseres Arbeiten mit dem Einführteil ermöglicht wird, was insbesondere in der Medizintechnik von großer Bedeutung ist. Genauso vorteilhaft wirkt sich die Verwendung eines erfindungsgemäßen Biegesensors in einem medizinischen Haltearm aus, bei dem wenigstens ein gelenkig ausgebildeter Abschnitt vorgesehen ist. Dabei ist der Biegesensor erfindungsgemäß in diesem Abschnitt angeordnet, um eine Auslenkung des Haltearms zu messen. Auch hier wird eine Rückmeldung über die tatsächliche Auslenkung und Lage des Haltearms ermöglicht, was dem Anwender erlaubt, den Haltearm verlässlich auszurichten, insbesondere unter Verwendung von Motoren. Selbstverständlich können auch mehrere gelenkige Abschnitte mit mehreren Biegesensoren vorgesehen sein. Denkbar ist auch eine Anwendung in der medizinischen Robotik, wo Biegesensoren zur Erfassung der Ausrichtung und Bewegung besonders wichtig sind. Da der erfindungsgemäße Biegesensor besonders leicht und kostengünstig herzustellen ist, kann auch problemlos eine größere Anzahl von Sensoren vorgesehen werden.

Die vorstehend genannten und die nachstehend noch zu erläuternden Merkmale sind nicht nur in der jeweils angegebenen Kombination, sondern auch in anderen Kombinationen oder in Alleinstellung verwendbar, ohne den Rahmen der vorliegenden Erfindung zu verlassen.

Die beschriebene Erfindung zeichnet sich durch einen langgestreckten flexiblen Biegesensor aus elektrisch isolierendem Kunststoff aus, in den längliche Fasern aus elektrisch leitendem Kunststoff eingebracht sind. Die Fasern verlaufen etwa parallel zur Längsachse des Sensors. Eine Biegung des Sensors führt zu einer Änderung der elektrischen Widerstände in den Fasern, die von einer Messapparatur erfasst wird. Diese ist zu diesem Zweck mit den Fasern des Sensors elektrisch verbunden. Die elektrische Schaltung der Fasern mit den Bauteilen der Messapparatur entspricht vorteilhafterweise einer Wheatstone'schen Brücke. Der erfindungsgemäße Biegesensor zeichnet sich durch einen kompakten und einfachen Aufbau aus, wobei eine Biegung zuverlässig und sicher erfasst wird. Es können weiterhin Mittel zur Versteifung des Sensors vorgesehen sein, die den Sensor formstabil gegen Verdrehung um die Längsachse machen.

Ausführungsbeispiele der Erfindung sind in den Zeichnungen näher dargestellt und werden in der nachfolgenden Beschreibung näher erläutert. Es zeigen:
- Fig. 1: eine Prinzipskizze des erfindungsgemäßen Biegesensors mit einer Messeinheit
- Fig. 2: den Biegesensor mit Flechtschlauch
- Fig. 3: den Biegesensor gemäß einem ersten Ausführungsbeispiel
- Fig. 4: den Biegesensor gemäß einem zweiten Ausführungsbeispiel
- Fig. 5: den Biegesensor gemäß einem dritten Ausführungsbeispiel
- Fig. 6: den Biegesensor mit Schrumpfschlauch
- Fig. 7: ein endoskopisches Instrument mit Biegesensor
- Fig. 8: das endoskopische Instrument mit Biegesensor im Querschnitt entlang der Linie A-A aus Figur 7
- Fig. 9: eine Prinzipskizze des Biegesensors in Verbindung mit einer Messeinheit und einer Motoreinheit
- Fig. 10: einen medizinischen Haltearm mit Biegesensor
- Fig. 11: den Biegesensor gemäß einem weiteren Ausführungsbeispiel.

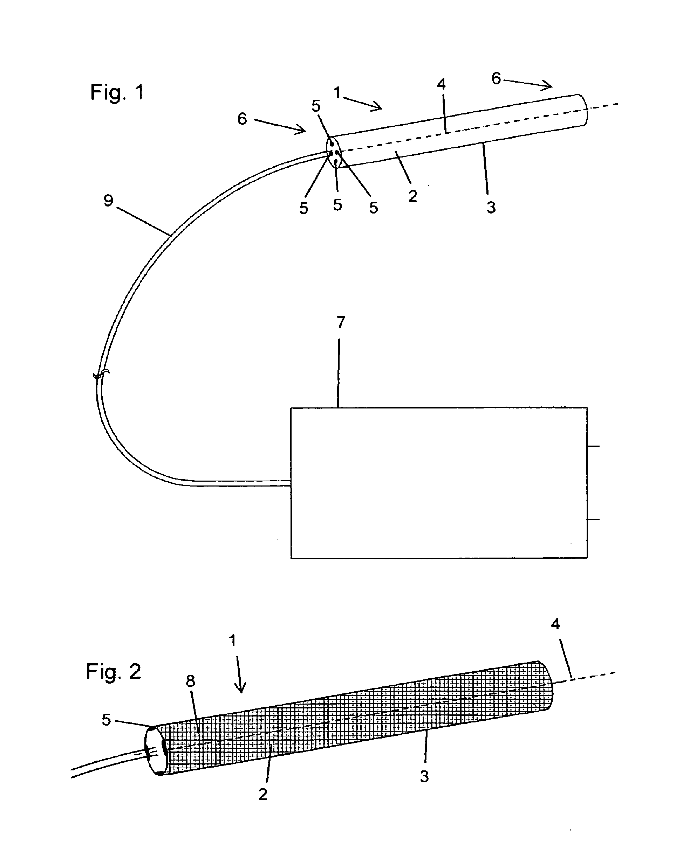

Figur 1 zeigt schematisch einen erfindungsgemäßen Biegesensor 1 mit einem Körper 2 aus elektrisch isolierendem Polymer und vier darin eingebetteten Fasern 5 aus elektrisch leitendem Polymer, die umfänglich um etwa 90° gegeneinander versetzt und voneinander beabstandet angeordnet sind. In den im Folgenden dargestellten Beispielen besteht der Körper aus einem Silikon, die Fasern bestehen aus einer elektrisch leitenden SilikonElastomer-Mischung. Die elektrische Leitfähigkeit des Kunststoffs wird beispielsweise durch die Beimischung von Nanopartikeln aus einem elektrisch leitenden Material wie Silber oder Graphit erreicht. Die Fasern 5 werden im Bereich der Faserenden 6 von elektrischen Leitungen 9 kontaktiert, die den Biegesensor 1 mit einer Messeinheit 7 verbinden. In Figur 1 ist beispielhaft nur eine der elektrischen Leitungen 9 dargestellt.

Wird der Biegesensor 1 gekrümmt und damit aus seiner Längsachse 4 ausgelenkt, so ändert sich der elektrische Widerstand in den leitenden Polymerfasern 5 mit dem Grad der Krümmung. Diese Änderung wird in der Messeinheit 7 erfasst. Dies wird später noch genauer dargestellt. Dabei ermöglichen zwei gegenüberliegende Fasern 5 die Bestimmung der Krümmung in einer Ebene, die zwei anderen Fasern 5 die Bestimmung der Krümmung in einer Ebene senkrecht dazu. Die tatsächliche Krümmung des Biegesensors 1 ergibt sich aus der Kombination dieser beiden Messungen. Auf diese Weise können Krümmungen erfindungsgemäß in alle Richtungen korrekt, einfach und sicher erfasst werden.

Der in Figur 2 dargestellte Biegesensor 1 ist entlang seiner Längsachse 4 von einem Flechtschlauch 8 umgeben, der mit der Oberfläche 3 des Körpers 2 verbunden ist. Insbesondere wird ein netzförmiger Flechtschlauch aus Nylon oder einem anderen Material mit Silikon- oder Polyurethan-Harz getränkt und auf den Körper 2 aufgebracht. Auf diese Weise wird ein Verdrehen des Körpers 2 um seine Längsachse beschränkt oder verhindert, welches die Messungen der Auslenkung verfälschen könnte. Die Fasern 5 verlaufen so vorzugsweise parallel zur Längsachse 4 des Körpers 2 des Biegesensors 1.

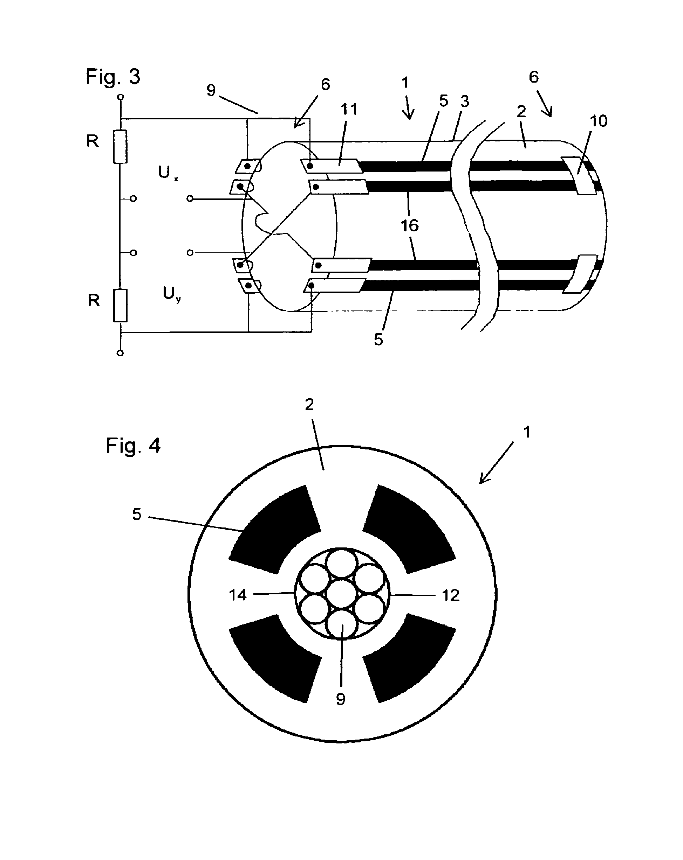

Die Figur 3 zeigt den Biegesensor gemäß einem ersten Ausführungsbeispiel, bei dem die vier Fasern 5 an der äußeren Oberfläche 3 des Körpers 2 angeordnet sind. Dies erlaubt eine direkte Verbindung der Kontakte 11 der elektrischen Leitungen 9 mit den Faserenden 6 auf der Außenseite des Körpers 2. Wie hier dargestellt ist, umfassen die elektrischen Leitungen 9 vier zusätzliche leitende Polymerfasern 16, die zusätzlich zu den Fasern 5 in den Körper 2 eingebettet sind und im Wesentlichen parallel zu diesen verlaufen. Auf diese Weise kann der Biegesensor mit den Fasern 5, die die veränderlichen Widerstände für die Messung der Auslenkung bilden, und den zusätzlichen Fasern 16 für die elektrische Leitung in einem Stück coextrudiert werden und so besonders schnell und einfach und in beliebiger Länge hergestellt werden. Es müssen keine Leitungen außen am Körper 2 des Biegesensors 1 entlang geführt werden, was den notwendigen Durchmesser des Biegesensors 1 weiter verringert.

Die elektrischen Leitungen 9 zur Verbindung mit der Messeinheit 7 greifen ausschließlich an einem Ende des Sensorkörpers 2 an, hier links dargestellt. Dies hat den Vorteil, dass alle Leitungen 9 in eine Richtung vom Sensor 1 weg zur Messeinheit 7 geführt werden können. Insbesondere bei Anwendung in einem Endoskop lassen sich so leicht alle Leitungen vom proximalen Ende des Biegesensors 1 zum proximalen Ende des Endoskops und zur Messeinheit 7 führen. Am anderen Ende, hier rechts dargestellt, muss lediglich eine Verbindung zwischen den Messfasern 5 und den zusätzlichen Fasern 16 sichergestellt werden. Dies kann über verschiedene dem Fachmann bekannte Maßnahmen geschehen, beispielsweise mit Mikroklammern aus Metall, die in das Polymermaterial gestochen werden und damit eine mechanisch und elektrisch sichere Verbindung schaffen. Besonders vorteilhaft ist jedoch die Verbindung über Flexprints 10, da diese besonders flach ausgebildet sind und eng um den Sensorkörper 2 angelegt werden können. Die auf den Flexprints 10 vorgesehenen Leiterbahnen stellen dann die elektrische Verbindung zwischen den verschiedenen Fasern 5 und 16 des Biegesensors 1 her.

Ebenfalls dargestellt ist die Verschaltung der Fasern 9 und der zusätzlichen Fasern 16 mit den Referenzwiderständen R zu einer Wheatstone'schen Brücke. Eine Änderung der elektrischen Widerstände der Fasern 5 gegenüber den Referenzwiderständen R, wie sie durch eine Biegung des Biegesensors 1 in den elektrisch leitenden Fasern 5 verursacht wird, führt zur Änderung einer Signalspannung Uₓ bzw. U_{y}, die einer Auslenkung des Biegesensors 1 in einer X- bzw. Y-Ebene entspricht. Die Signalspannungen Uₓ bzw. U_{y} geben Auskunft über das Maß der Auslenkung aus der Längsachse 4. Sie geben außerdem die X- und die Y-Komponente der Auslenkung an. Die Referenzwiderstände können beispielsweise in der Messeinheit 7 angeordnet sein, die auch die Signalspannungen erfasst und auswertet. Die Referenzwiderstände können gleich oder unterschiedlich groß sein, es können die gleichen Widerstände für beide Richtungen als Referenz dienen oder für die X- und Y-Messung unterschiedliche Referenzwiderstände vorgesehen sein.

Die zusätzlichen Fasern 16 können auch durch eigene Widerstandsänderung bei der Biegung zum Messsignal beitragen.

Es ist auch möglich, durch geeignete elektrische Verschaltung der leitenden Fasern 5 und 16 einen erfindungsgemäßen Biegesensor mit nur zwei zusätzlichen Polymerfasern 16 zu realisieren, also mit insgesamt sechs leitenden Fasern.

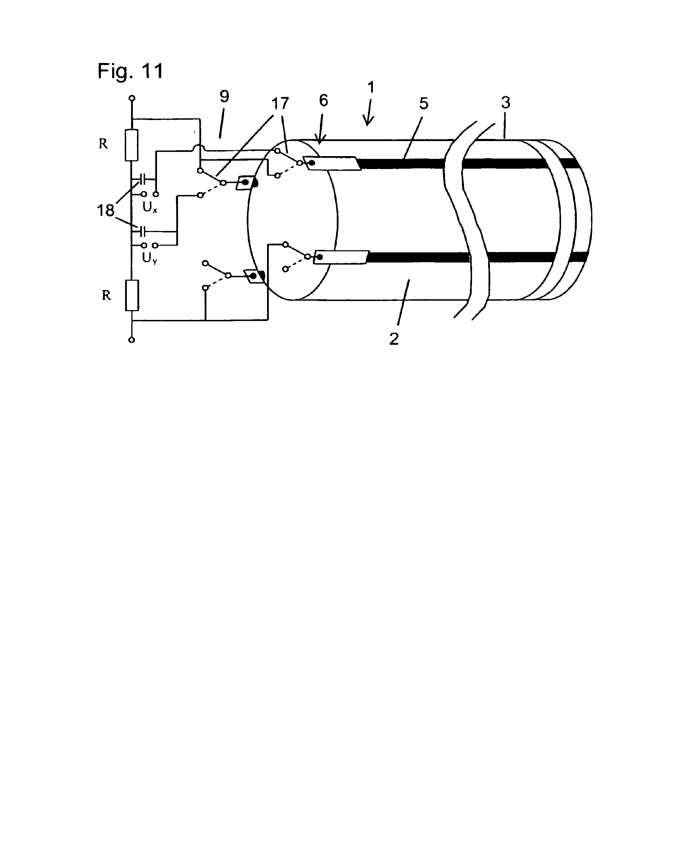

In einem weiteren in Figur 11 dargestellten Ausführungsbeispiel des Biegesensors 1 sind nur vier leitende Fasern 5 vorgesehen, die einmal durch ihren bei Biegung veränderlichen Widerstand der Messung der Auslenkung aus der Längsachse 4 dienen und einmal der elektrischen Verbindung mit den elektrischen Zuleitungen 9 bzw. als Teil dieser Zuleitungen. Hierfür ist der Sensor 1 am Kontaktierungsende (links dargestellt) an den Faserenden 6 jeweils mit Umschaltern 17 verbunden, vorzugsweise mit elektronisch gleichgetakteten Halbleiterschaltern, die auch untereinander verknüpft sind. In einer ersten Stellung der Umschalter 17 fliesst der Strom beispielsweise durch jene Fasern 5, die der Messung der Auslenkung entlang der X-Achse dienen. Die elektrische Verbindung dieser Messfasern mit den Zuleitungen 9 erfolgt dann über wenigstens eine in dieser Stellung nicht für die Messung genutzte Faser, die der Erfassung der Auslenkung in Y-Richtung zugeordnet ist. In einer zweiten Stellung der Umschalter 17, hier gestrichelt dargestellt, wird analog die Widerstandsänderung der für die Messung der Y-Auslenkung vorgesehenen Fasern erfasst und wenigstens eine Faser, die der Messung in X-Richtung zugeordnet ist, als elektrische Leitung 9 verwendet. Durch permanentes schnelles Umschalten zwischen der ersten und der zweiten Stellung können beide Richtungskomponenten X und Y der Auslenkung mit Hilfe von Kondensatoren 18 nahezu gleichzeitig gemessen werden.

Am den Umschaltern 17 entgegengesetzten Ende des Biegesensors 1 werden die Fasern 5 auf einfache Weise wie in den anderen Ausführungsbeispielen beschrieben miteinander elektrisch verbunden um die Schaltung zu vervollständigen.

Die Auslenkung kann durch geeignete Schaltung als separate Spannung Uₓ und U_{y} jeweils für die X- und Y-Komponente der Biegung erfasst werden oder auch als eine Spannung U gemessen werden, die während des permanenten Umschaltens in der ersten Stellung der Spannung Uₓ und in der zweiten Stellung der Spannung U_{y} entspricht.

Durch diesen besonders einfachen Aufbau des Biegesensors 1 reduziert sich die Zahl der benötigten Fasern 5, was die Herstellung vereinfacht.

Die Fig. 4 zeigt den Querschnitt eines Biegesensors 1 gemäß einem weiteren Ausführungsbeispiel. Hier sind die Polymerfasern 5 vollständig vom isolierenden Körper 2 umschlossen. Der Sensor 1 hat hier einen runden Querschnitt, was eine leichte und gleichmäßige Auslenkung aus der Längsachse in alle Richtungen ermöglicht. Die elektrischen Leitungen 9, die an den nicht dargestellten Faserenden mit den Fasern 5 verbunden sind, werden hier in Form von Drähten wenigstens teilweise in einem Kanal 12 geführt, der etwa zentral entlang der Längsachse 4 durch den Biegesensor 1 hindurch verläuft. Auf diese Weise vergrößern die elektrischen Leitungen 9 nicht den Querschnitt des Biegesensors 1. Die Verbindung der elektrischen Leitungen 9 mit den Faserenden ist beispielsweise dadurch geschehen, dass die Drähte 9 den Körper 2 und die Fasern 5 je nach gewünschter elektrischer Verschaltung durchstechen. Da es mit der Zeit materialbedingt zu einer Alterung oder einer Verformung des Körpers 2 kommen kann, ist der Kanal 12 an seinen Enden jeweils mit einem Röhrchen 14 zur Verstärkung versehen. Die Röhrchen 14 verhindern eine Veränderung des Kanaldurchmessers durch die Materialermüdung oder durch ein Kriechen des Materials. Diese Röhrchen können mit einer Isolierung versehen sein, um eine elektrische Isolierung gegen die elektrischen Leitungen 9 sicherzustellen.

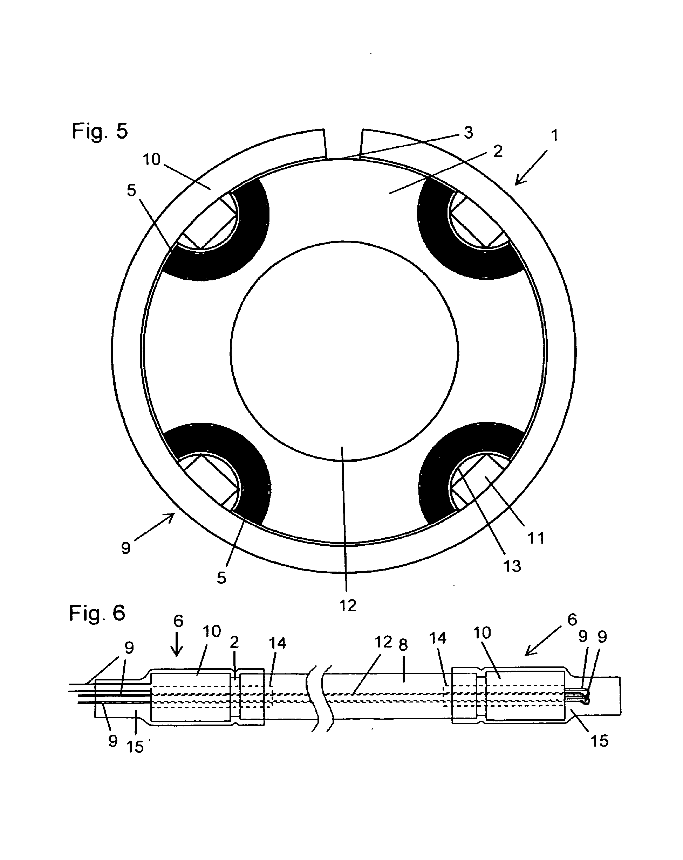

Der in Figur 5 im Querschnitt dargestellte Biegesensor 1 gemäß einem weiterenAusfüh-rungsbeispiel umfasst wieder einen Körper 2 mit einem mittigen, sich in Längsrichtung erstreckenden Kanal 12, durch den ein Teil der elektrischen Leitungen 9 geführt werden kann. Die leitenden Polymerfasern 5 liegen hier teilweise an der Oberfläche des Körpers 2 und werden von außen angelegten Flexprints 10 kontaktiert. Die Flexprints 10 sind dem Durchmesser des Körpers 2 entsprechend vorgeformt und umschließen den Körper 2 eng anliegend, um den Durchmesser des Biegesensors 1 nicht unnötig zu vergrößern. Die Flexprints 10 weisen Kontakte 11 in Form von Vorsprüngen auf, die in Vertiefungen 13 in den leitenden Fasern 5 eingreifen. Es ist auch denkbar die Fasern 5 wenigstens im Bereich der Faserenden 6 mit solchen Vorsprüngen zu versehen, die umgekehrt in Vertiefungen in den Kontakten 11 eingreifen. Auch eine Kombination von beidem ist möglich. In jedem Fall wird eine sichere Kontaktierung gewährleistet und ein Verrutschen der Kontakte verhindert.

Die Fig. 6 zeigt eine seitliche Darstellung des Biegesensors 1. Der Körper 2 ist über seine Länge von einem Flechtschlauch 8 in Form einer mit dem Sensor coextrudierten Flechtschicht umgeben. Im Bereich der Faserenden 6 wird der Biegesensor wie im vorhergehenden Ausführungsbeispiel erläutert von Flexprints 10 kontaktiert. Die weiteren elektrischen Leitungen 9, die zur Verbindung der elektrisch leitenden Polymerfasern 5 mit der hier nicht dargestellten Messeinheit 7 dienen, verlaufen durch den mittigen Kanal 12 vom hier rechts dargestellten Ende des Biegesensors 1 zum linken Ende des Biegesensors 1 und weiter zur Messeinheit 7. Im dargestellten Beispiel handelt es sich bei den weiteren elektrischen Leitungen 9 um Fortsätze der Flexprints 10. Der Kanal 12 ist an seinen Enden jeweils mit einem Röhrchen 14 verstärkt. Um einen sicheren Halt der Flexprints 10 zu gewährleisten, ist der Sensor im Bereich seiner Enden jeweils zusätzlich mit einem Schrumpfschlauch 15 versehen, der den Körper 2, das Netzgeflecht 8 und die Flexprints 10 fest umschließt.

Dadurch ist eine sichere Anordnung geschaffen, die auch unter schwierigen Außenbedingungen verlässliche Informationen zur Auslenkung des Sensors 1 liefert.

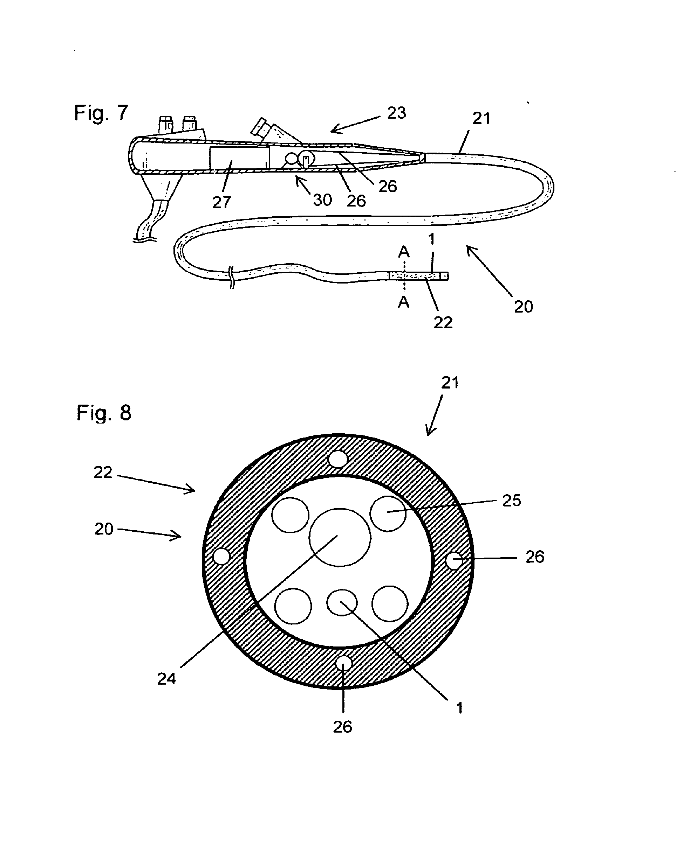

Das in Figur 7 dargestellte endoskopische Instrument ist ein flexibles Endoskop 20 und umfasst einen Einführteil 21 mit einem flexiblen Abschnitt 22, sowie ein proximales Gehäuse 23. In diesem Beispiel ist der flexible Abschnitt 22 am distalen Ende des Einführteils 21 angeordnet. Es ist jedoch auch denkbar, dass einer oder mehrere flexible Abschnitte über den Einführteil verteilt vorgesehen sind. Die Bowdenzüge 26 verlaufen vom Inneren des Gehäuses 23 durch den Einführteil 21 zum flexiblen Abschnitt 22. Durch die Bewegung einer Mechanik 30 im Gehäuse 23 wird der flexible Abschnitt 22 mittels der Bowdenzüge 26 ausgelenkt. Dies kann mit der Hand durch Betätigung eines nicht dargestellten, am Gehäuse 23 angebrachten Stellrades geschehen, oder auch elektrisch über eine Motoreinheit 27. Hier sind die Bowdenzüge 26 über die Mechanik 30 mit der Motoreinheit 27 verbunden.

Das flexible Endoskop 20 umfasst außerdem einen Biegesensor 1 nach einem der vorhergehend beschriebenen Beispiele, der im Bereich des flexiblen Abschnitts 22 angeordnet ist. Bei Vorhandensein mehrerer flexibler Abschnitte ist es sinnvoll, jeden Abschnitt mit einem eigenen Biegesensor zu versehen. Wird nun der flexible Abschnitt 22 ausgelenkt, so wird die Auslenkung vom Biegesensor 1 erfasst und ein entsprechendes Signal an die hier nicht dargestellte Messeinheit 7 weitergeleitet. Die Messeinheit 7 kann sich hierzu außerhalb des endoskopischen Instruments 20 befinden oder auch im Instrument 20 integriert sein, beispielsweise im Gehäuse 23.

Die Figur 8 zeigt einen Querschnitt durch den Einführteil 21 des flexiblen Endoskops 20 im Bereich des flexiblen Abschnitts 22 entlang der Linie A-A aus Figur 7.

Der Einführteil 21 dieses Instruments 20 weist in seinem Querschnitt einen Bildleiter 24 auf, der ein distal empfangenes Lichtsignal nach proximal weiterleitet, sowie Lichtleiterbündel 25, die der Weiterleitung von Beleuchtungslicht vom proximalen an das distale Ende dienen. Im äußeren Bereich des Querschnitts sind die Bowdenzüge 26 dargestellt. Um für eine gleichmäßige Ausleuchtung des zu betrachtenden Objekts zu sorgen, sind meist mehrere Lichtleiterbündel 25 im Querschnitt vorgesehen. Insbesondere in der Medizintechnik beträgt der Durchmesser der flexiblen Endoskopschäfte bevorzugt nur wenige Millimeter, so dass nur wenig Raum für weitere Komponenten im Einführteil zur Verfügung steht. Der erfindungsgemäße Biegesensor 1, der wie in einem der vorhergehend genannten Beispiele ausgebildet ist, ist zwischen den anderen Komponenten Bildleiter 24 und Lichtleiter 25 im Einführteil 21 angeordnet und von diesem vollständig umschlossen. Aufgrund seiner kompakten Bauweise und seines kleinen Durchmessers von weniger als einem Millimeter ist die Integration in den Einführteil 21 des Endoskops möglich. Der Raum proximal des Biegesensors 1 kann genutzt werden, um die elektrischen Leitungen 9 unterzubringen und zum proximalen Ende des Instruments 20 zu führen, was zu einem kompakten Gesamtaufbau des flexiblen Endoskops 20 beiträgt.

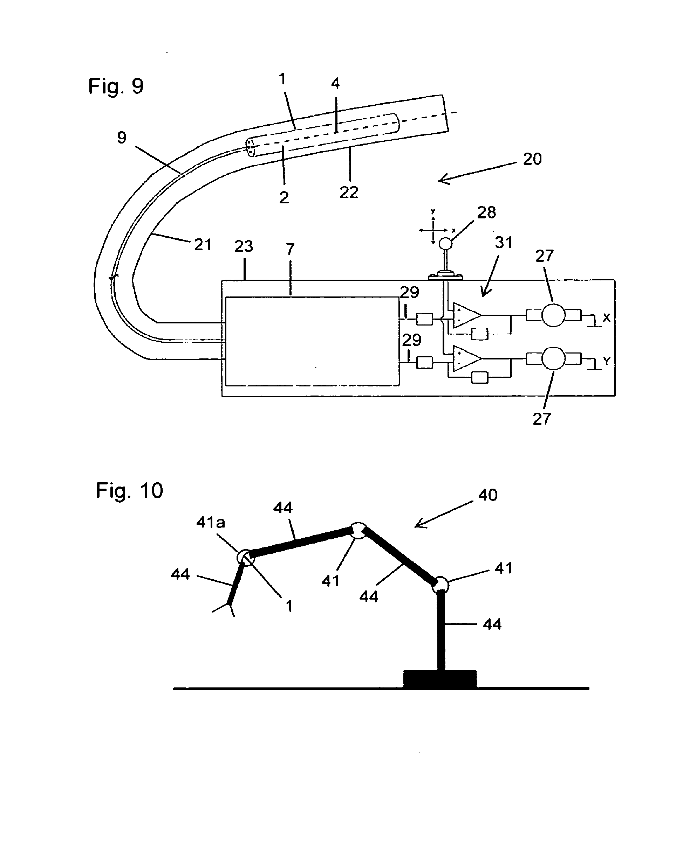

Die Figur 9 verdeutlicht den Zusammenhang zwischen Biegesensor 1 und Messeinheit 7 bzw. Motoreinheiten 27, wie er in einem flexiblen Endoskop 20 vorgesehen sein kann. Wie zuvor zu Figur 7 beschrieben, befindet sich der Biegesensor 1 im flexiblen Abschnitt 22 des Einführteils 21 des Endoskops 20. Im proximalen Gehäuse 23 ist nun die Messeinheit 7 vorgesehen. Signalleitungen 29 verbinden die Messeinheit 7 mit einer Steuerung 31, die einerseits mit einem Joystick 28 und andererseits mit den Motoreinheiten 27 verbunden ist. Durch Betätigung des Joysticks 28 werden über die Steuerung 31 die Motoreinheiten 27 dazu veranlasst über die hier nicht dargestellte Mechanik 30 die Bowdenzüge 26 zu bewegen. Auf diese Weise wird der flexible Abschnitt 22 des Einführteils 21 ausgelenkt.

Da der Biegesensor 1 mit ausgelenkt wird, erzeugt er ein Signal das die tatsächliche Biegung des Abschnitts 22 repräsentiert und das an die Messeinheit 7 übertragen wird. Diese wertet das Signal aus und leitet ein adaptiertes Steuersignal weiter an die Steuerung 31. Auf diese Weise werden mechanische Unzulänglichkeiten, wie sie in flexiblen Endoskopen beispielsweise durch die vorhandene Seilreibung auftreten, automatisch kompensiert. Dadurch wird erreicht, dass die Betätigung des Joysticks 28 genau mit der Bewegung des flexiblen Abschnitts 22 korreliert. Auftretende mechanische Beeinflussungen werden während der Anwendung kompensiert, da der Biegesensor 1 fortwährend genaue Informationen über den tatsächlichen Auslenkungsgrad des flexiblen Abschnitts 22 an die Messeinheit 7 liefert.

Ein erfindungsgemäßer Haltearm 40 ist in Figur 10 dargestellt. Ein solcher medizinischer Haltearm 40 dient beispielsweise dem Halten medizinischer Instrumente in einem Operationssaal. Er umfasst in diesem Beispiel drei gelenkige Abschnitte 41, 41 a, die es ermöglichen die verschiedenen Teile 44 des Haltearms gegeneinander auszulenken. Im gelenkigen Abschnitt 41 a ist ein Biegesensor 1 nach einem der vorher genannten Ausführungsbeispiele vorgesehen. Er erfasst zuverlässig eine Auslenkung der durch den gelenkigen Abschnitt miteinander verbundenen Teile 44 des Haltearms 40. Es versteht sich, dass eine beliebige Zahl von gelenkigen Abschnitten 41, 41a mit einem erfindungsgemäßen Biegesensor 1 versehen werden kann, um eine Ausrichtung des Haltearms 40 insgesamt präzise zu erfassen.

## Patentansprüche

1. Biegesensor (1) zur Messung einer Auslenkung
bestehend aus einem lang erstreckten Körper (2) aus elektrisch isolierendem Polymermaterial,
mit einer Längsachse (4),
mit in den Körper (2) eingebetteten Fasern (5) aus einem elektrisch leitenden Polymermaterial,
wobei die Fasern (5) im Wesentlichen parallel zur Längsachse (4) und voneinander beabstandet im Polymerkörper (2) angeordnet sind, wobei die Fasern (5) durch den Körper (2) voneinander getrennt sind,
**dadurch gekennzeichnet,**
**dass** eine Messeinheit (7) vorgesehen ist, die mit den Fasern (5) verbunden ist und geeignet ist zur Bestimmung der Änderung der elektrischen Widerstände der Fasern (5) als Maß der Auslenkung des Körpers (2) aus der Längsachse (4).

2. Biegesensor (1) nach Anspruch 1, **dadurch gekennzeichnet, dass** wenigstens vier Fasern (5) im Körper umfänglich um etwa 90° gegeneinander versetzt angeordnet sind.

3. Biegesensor (1) nach Anspruch 1 oder 2, **dadurch gekennzeichnet, dass** Mittel zur Versteifung des Sensors (1) vorgesehen sind, die eine Verdrehung des Biegesensors (1) um seine Längsachse (4) beschränken.

4. Biegesensor (1) nach Anspruch 3, **dadurch gekennzeichnet, dass** der Körper (2) entlang seiner Längsachse (4) von einem am Körper (2) anliegenden Flechtschlauch (8) umschlossen ist.

5. Biegesensor (1) nach einem der vorherigen Ansprüche, **dadurch gekennzeichnet, dass** sein Durchmesser über seine ganze Länge im Wesentlichen kleiner als 1 mm ist.

6. Biegesensor (1) nach einem der vorherigen Ansprüche, **dadurch gekennzeichnet, dass** elektrische Leitungen (9, 16) vorgesehen sind, die die Fasern (5) im Bereich der Faserenden (6) kontaktieren.

7. Biegesensor (1) nach einem der vorherigen Ansprüche, **dadurch gekennzeichnet, dass** die Fasern (5) wenigstens teilweise an der Oberfläche (3) des Körpers (2) liegen.

8. Biegesensor (1) nach einem der vorherigen Ansprüche, **dadurch gekennzeichnet, dass** jeweils zwei der Fasern (5) und die elektrischen Leitungen (9, 16) zusammen mit der Messeinheit (7) eine elektrische Schaltung in Form einer Wheatstone'schen Brücke bilden.

9. Biegesensor (1) nach einem der vorherigen Ansprüche, **dadurch gekennzeichnet, dass** die elektrischen Leitungen (9, 16) wenigstens teilweise in das elektrisch isolierende Polymermaterial des Körpers (2) eingebettet sind.

10. Biegesensor (1) nach Anspruch 9, **dadurch gekennzeichnet, dass** die elektrischen Leitungen (9, 16) zusätzliche in den Körper (2) eingebettete Fasern (16) aus dem elektrisch leitenden Polymermaterial umfassen.

11. Biegesensor (1) nach einem der vorherigen Ansprüche, **dadurch gekennzeichnet, dass** der Körper (2) einen insbesondere zentralen Kanal (12) entlang seiner Längsachse (4) aufweist, durch den wenigstens ein Teil der elektrischen Leitungen (9) geführt ist.

12. Biegesensor (1) nach einem der vorherigen Ansprüche, **dadurch gekennzeichnet, dass** die elektrischen Leitungen (9) Flexprints (10) mit elektrischen Kontakten (11) umfassen, die am Körper (2) anliegen und die Fasern (5) kontaktieren.

13. Biegesensor (1) nach Anspruch 12, **dadurch gekennzeichnet, dass** die Fasern (5,16) und/oder die Flexprints (10) Vertiefungen (13) aufweisen, in welche Vorsprünge der jeweils anderen Komponente Flexprint (10) oder Faser (5) eingreifen.

14. Biegesensor (1) nach einem der vorherigen Ansprüche, **dadurch gekennzeichnet, dass** ein Schrumpfschlauch (15) vorgesehen ist, der den Biegesensor (1) wenigstens teilweise umschließt.

15. Biegesensor (1) nach einem der vorherigen Ansprüche, **dadurch gekennzeichnet, dass** er in ein medizinisches oder endoskopisches Instrument (20) mit einem Einführteil (21) integriert ist, der wenigstens einen flexiblen Abschnitt (22) aufweist, und dass der Biegesensor (1) im Bereich des flexiblen Abschnitts (22) im Einführteil (21) angeordnet ist, um eine Auslenkung des Einführteils (21) zu messen.

16. Biegesensor (1) nach Anspruch 15, **dadurch gekennzeichnet, dass** der Biegesensor (1) vollständig vom Einführteil (21) umschlossen ist.

17. Biegesensor (1) nach Anspruch 15 oder 16, **dadurch gekennzeichnet, dass** wenigstens eine Motoreinheit (27) vorgesehen ist, die mit dem flexiblen Abschnitt (22) des Instruments (20) in Wirkverbindung steht, um eine Auslenkung des Einführteils (21) zu bewirken, wobei die Messeinheit (7) und die Motoreinheit (27) über Signalleitungen (29) zur Übertragung eines Signals als Maß der Auslenkung miteinander verbunden sind.

18. Biegesensor (1) nach einem der Ansprüche 1 bis 14, **dadurch gekennzeichnet, dass** er in einen medizinischen Haltearm (40) mit wenigstens einem gelenkig ausgebildeten Abschnitt (41, 41a) integriert ist, wobei der Biegesensor (1) in diesem Abschnitt (41a) angeordnet ist, um eine Auslenkung des Haltearms (40) zu messen.

## Claims

1. Bend sensor (1) for measuring a deflection,
consisting of an elongated body (2) of electrically insulating polymer material, with a longitudinal axis (4),
with fibres (5) of an electrically conducting polymer material embedded in the body (2),
wherein the fibres (5) are disposed essentially parallel to the longitudinal axis (4) and at a distance from one another in the polymer body (2), wherein the fibres (5) are separated from one another by the body (2),
**characterized**
**in that** a measuring unit (7) that is connected to the fibres (5) and is suitable for determining the changing of the electrical resistances of the fibres (5) as a measure of the deflection of the body (2) from the longitudinal axis (4) is provided.

2. Bend sensor (1) according to Claim 1, **characterized in that** at least four fibres (5) are disposed in the body, peripherally offset by about 90° from one another.

3. Bend sensor (1) according to Claim 1 or 2, **characterized in that** means for stiffening the sensor (1) that restrict rotation of the bend sensor (1) about its longitudinal axis (4) are provided.

4. Bend sensor (1) according to Claim 3, **characterized in that** the body (2) is enclosed along its longitudinal axis (4) by a braided tube (8) lying against the body (2).

5. Bend sensor (1) according to one of the preceding claims, **characterized in that** its diameter is essentially less than 1 mm over its entire length.

6. Bend sensor (1) according to one of the preceding claims, **characterized in that** electrical lines (9, 16) that contact the fibres (5) in the area of the fibre ends (6) are provided.

7. Bend sensor (1) according to one of the preceding claims, **characterized in that** the fibres (5) lie at least partially against the surface (3) of the body (2).

8. Bend sensor (1) according to one of the preceding claims, **characterized in that** two of the fibres (5) and the electrical lines (9, 16) respectively form together with the measuring unit (7) an electrical circuit in the form of a Wheatstone bridge.

9. Bend sensor (1) according to one of the preceding claims, **characterized in that** the electrical lines (9, 16) are at least partially embedded in the electrically insulating polymer material of the body (2).

10. Bend sensor (1) according to Claim 9, **characterized in that** the electrical lines (9, 16) comprise additional fibres (16) of the electrically conducting polymer material that are embedded in the body (2).

11. Bend sensor (1) according to one of the preceding claims, **characterized in that** the body (2) has along its longitudinal axis (4) a channel (12), in particular a central channel (12), through which at least some of the electrical lines (9) are led.

12. Bend sensor (1) according to one of the preceding claims, **characterized in that** the electrical lines (9) comprise flex prints (10) with electrical contacts (11) that lie against the body (2) and contact the fibres (5).

13. Bend sensor (1) according to Claim 12, **characterized in that** the fibres (5, 16) and/or the flex prints (10) have recesses (13) in which protrusions of the respectively other component, flex print (10) or fibre (5), engage.

14. Bend sensor (1) according to one of the preceding claims, **characterized in that** a shrink tube (15) that at at least partially encloses the bend sensor (1) is provided.

15. Bend sensor (1) according to one of the preceding claims, **characterized in that** it is integrated in a medical or endoscopic instrument (20) with an insertion part (21) that has at least one flexible portion (22), and **in that** the bend sensor (1) is disposed in the area of the flexible portion (22) in the insertion part (21) in order to measure a deflection of the insertion part (21).

16. Bend sensor (1) according to Claim 15, **characterized in that** the bend sensor (1) is completely enclosed by the insertion part (21).

17. Bend sensor (1) according to Claim 15 or 16, **characterized in that** at least one motor unit (27) that is operatively connected to the flexible portion (22) of the instrument (20) in order to cause a deflection of the insertion part (21) is provided, wherein the measuring unit (7) and the motor unit (27) are connected to one another via signal lines (29) to transmit a signal as a measure of the deflection.

18. Bend sensor (1) according to one of Claims 1 to 14, **characterized in that** it is integrated in a medical holding arm (40) with at least one jointly configured portion (41, 41a), wherein the bend sensor (1) is disposed in this portion (41a) in order to measure a deflection of the holding arm (40).

## Revendications

1. Capteur de flexion (1) mesurant une déformation, constitué d'un corps allongé (2) en matériau polymère électriquement isolant qui présente
un axe longitudinal (4),
des fibres (5) en matériau polymère électriquement conducteur incorporées dans le corps (2),
les fibres (5) étant disposées essentiellement parallèlement à l'axe longitudinal (4) et à distance les unes des autres dans le corps polymère (2),
les fibres (5) étant séparées les unes des autres par le corps (2),
**caractérisé en ce que**
une unité de mesure (7) raccordée aux fibres (5) et convenant pour déterminer la modification de la résistance électrique des fibres (5) comme mesure de la déformation du corps (2) par rapport à l'axe longitudinal (4) est prévue.

2. Capteur de flexion (1) selon la revendication 1, **caractérisé en ce qu'**au moins quatre fibres (5) sont disposées à la périphérie dans le corps à un décalage mutuel d'environ 90°.

3. Capteur de flexion (1) selon les revendications 1 ou 2, **caractérisé en ce que** des moyens de rigidification du capteur (1) sont prévus pour limiter une rotation du capteur de flexion (1) autour de son axe longitudinal (4).

4. Capteur de flexion (1) selon la revendication 3, **caractérisé en ce que** le corps (2) est englobé par un tuyau flexible tressé (8) placé sur le corps (2) le long de son axe longitudinal (4).

5. Capteur de flexion (1) selon l'une des revendications précédentes, **caractérisé en ce que** son diamètre est essentiellement inférieur à 1 mm sur toute sa longueur.

6. Capteur de flexion (1) selon l'une des revendications précédentes, **caractérisé en ce que** des conducteurs électriques (9, 16) qui assurent le contact avec les fibres (5) sont prévus au niveau des extrémités (6) des fibres.

7. Capteur de flexion (1) selon l'une des revendications précédentes, **caractérisé en ce que** les fibres (5) sont situées au moins en partie à la surface (3) du corps (2).

8. Capteur de flexion (1) selon l'une des revendications précédentes, **caractérisé en ce que** deux des fibres (5) et les conducteurs électriques (9, 16) forment avec l'unité de mesure (7) un circuit électrique qui présente la forme d'un pont de Wheatstone.

9. Capteur de flexion (1) selon l'une des revendications précédentes, **caractérisé en ce que** les conducteurs électriques (9, 16) sont incorporés au moins en partie dans le matériau polymère électriquement isolant du corps (2).

10. Capteur de flexion (1) selon la revendication 9, **caractérisé en ce que** les conducteurs électriques (9, 16) comprennent des fibres supplémentaires (16) en matériau polymère électriquement conducteur incorporées dans le corps (2).

11. Capteur de flexion (1) selon l'une des revendications précédentes, **caractérisé en ce que** le corps (2) présente le long de son axe longitudinal (4) un canal (12) en particulier central dans lequel une partie des conducteurs électriques (9) est passée.

12. Capteur de flexion (1) selon l'une des revendications précédentes, **caractérisé en ce que** les conducteurs électriques (9) comprennent des impressions flexibles (10) dotées de contacts électriques (11) qui reposent contre le corps (2) et qui sont en contact avec les fibres (5).

13. Capteur de flexion (1) selon la revendication 12, **caractérisé en ce que** les fibres (5, 16) et/ou les impressions flexibles (10) présentent des creux (13) dans lesquels des saillies de l'autre composant engagent des impressions flexibles (10) ou des fibres (5).

14. Capteur de flexion (1) selon l'une des revendications précédentes, **caractérisé en ce qu'**un flexible rétrécissable (15) est prévu pour entourer au moins en partie le capteur de flexion (1).

15. Capteur de flexion (1) selon l'une des revendications précédentes, **caractérisé en ce qu'**il est intégré dans un instrument médical ou endoscopique (20) doté d'une partie d'insertion (21) qui présente au moins une partie flexible (22) et **en ce que** le capteur de flexion (1) est disposé dans la partie d'insertion (21) au niveau de la partie flexible (22) pour mesurer la déformation de la partie d'insertion (21).

16. Capteur de flexion (1) selon la revendication 15, **caractérisé en ce que** le capteur de flexion (1) est entièrement englobé par la partie d'insertion (21).

17. Capteur de flexion (1) selon les revendications 15 ou 16, **caractérisé en ce qu'**au moins une unité de moteur (27) qui coopère avec la partie flexible (22) de l'instrument (20) pour obtenir une déformation de la partie d'insertion (21) est prévu, l'unité de mesure (7) et l'unité de moteur (27) étant raccordées l'une à l'autre par des conducteurs (29) de signaux qui transmettent un signal qui sert de mesure de la déformation.

18. Capteur de flexion (1) selon l'une des revendications 1 à 14, **caractérisé en ce qu'**il est intégré dans un bras médical de maintien (40) doté d'au moins une partie (41, 41a) articulée, le capteur de flexion (1) étant disposé dans cette partie (41a) pour mesurer une déformation du bras de maintien (40).
